(19) **Europäisches Patentamt / European Patent Office / Office européen des brevets**

(11) **EP 2 865 420 B1**

(12) **FASCICULE DE BREVET EUROPEEN**

(45) Date de publication et mention de la délivrance du brevet:
**19.09.2018 Bulletin 2018/38**

(51) Int Cl.:
***A61N 7/02*** *(2006.01)*

(21) Numéro de dépôt: **14189686.0**

(22) Date de dépôt: **21.10.2014**

(54) **Appareil de génération d'ondes ultrasonores focalisées à temps de traitement réduit**

Gerät zur Erzeugung von fokalisierten Ultraschallwellen mit reduzierter Verarbeitungszeit

Apparatus for generating focused ultrasonic waves with reduced treatment time

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.10.2013 FR 1360343**

(43) Date de publication de la demande:
**29.04.2015 Bulletin 2015/18**

(73) Titulaires:
- **EDAP TMS France**
  **69120 Vaulx-en-Velin (FR)**
- **I.N.S.E.R.M. Institut National de la Santé et de la Recherche Médicale**
  **75654 Paris Cedex 13 (FR)**

(72) Inventeurs:
- **Brasset, Lucie**
  **69100 Villeurbanne (FR)**
- **Chapelon, Jean-Yves**
  **69100 Villeurbanne (FR)**
- **Guillen, Nicolas**
  **69270 Fontaine-Sur-Saone (FR)**
- **Chavrier, Françoise**
  **38300 Chezeneuve (FR)**
- **Blanc, Emmanuel**
  **69370 Saint Didier Au Mont D'Or (FR)**

(74) Mandataire: **Thibault, Jean-Marc et al
Cabinet Beau de Loménie
51, Avenue Jean Jaurès
B.P. 7073
69301 Lyon Cedex 07 (FR)**

(56) Documents cités:
WO-A1-2010/127369    WO-A2-2009/050719
DE-T2- 69 417 639    US-A- 5 665 054
US-A1- 2011 144 544   US-A1- 2013 041 249
US-A1- 2013 261 461   US-B1- 6 488 639

- JOSHUA COON ET AL: "HIFU treatment time reduction in superficial tumours through focal zone path selection", INTERNATIONAL JOURNAL OF HYPERTHERMIA, BASINGSTOKE, GB, vol. 27, no. 5, 1 août 2011 (2011-08-01), pages 465-481, XP008170207, ISSN: 0265-6736, DOI: 10.3109/02656736.2011.564597

**Description**

La présente invention concerne le domaine technique des appareils ou des dispositifs comportant une sonde ultrasonore adaptée pour émettre dans une zone focale, des ultrasons focalisés à haute intensité (HIFU).

L'objet de l'invention trouve des applications particulièrement avantageuses dans le domaine des traitements thérapeutiques par des ondes ultrasonores focalisées.

De manière classique, le principe des ultrasons thérapeutiques consiste à focaliser un faisceau ultrasonore émis par une sonde ultrasonore, sur une zone cible dans des tissus biologiques du corps humain par exemple. Les ultrasons se propagent de la sonde vers le point de focalisation en traversant les tissus biologiques, une partie de leur énergie étant absorbée par ces tissus et convertie en chaleur. L'élévation de température a lieu préférentiellement dans la zone de focalisation des ultrasons (volume focal), là où l'amplitude et l'intensité des ondes ultrasonores est maximale. Lorsque l'élévation de température et le temps pendant lequel les tissus sont soumis à cette température dépassent un certain seuil (dose thermique), il se produit une destruction irréversible des tissus. Les tissus situés entre la zone de focalisation et la sonde ultrasonore sont soumis à un échauffement largement moins intense et la vascularisation naturelle permet d'évacuer l'apport thermique de telle sorte que ces tissus ne subissent aucun dommage.

Cette technique est intéressante dans la mesure où elle permet de détruire des tissus biologiques par abord extracorporel ou endocavitaire, c'est-à-dire sans pratiquer d'incision chirurgicale. Le principe de la thérapie par ultrasons focalisés est appliqué par exemple au traitement de tumeurs malignes de la prostate, du foie ou du rein mais également au traitement de tumeurs bénignes telles que le fibrome utérin par exemple. Le brevet EP 1 858 591 décrit cette technique thérapeutique dans le cadre du traitement du cancer de la prostate.

La durée des traitements est reconnue comme un facteur très limitatif des applications des thérapies ultrasonores focalisées. Ainsi, ces traitements s'adressent plutôt à de petits organes, ou de petites tumeurs dans des organes. La durée des traitements résulte essentiellement de deux effets :

- l'effet de focalisation qui fait que le faisceau ultrasonore converge dans un volume typiquement de quelques mm$^3$ seulement. Pour couvrir tout un organe ou la totalité d'une tumeur, il faut alors répéter les impulsions ultrasonores et les juxtaposer. Plusieurs centaines d'impulsions sont alors nécessaires. Chacune d'elles peut durer plusieurs secondes et le traitement lui-même peut durer une heure ou plus selon le volume de traitement ;
- la répétition des impulsions ultrasonores provoque une accumulation de dépôt de chaleur dans les tissus situés entre la sonde ultrasonore et la zone de focalisation. Pour laisser le temps à la vascularisation naturelle de dissiper cette accumulation de chaleur, il convient d'aménager un temps d'attente entre chaque impulsion, temps pendant lequel l'émission est interrompue. Ce temps d'attente vient encore allonger la durée du traitement. Le rapport du temps d'émission de l'impulsion ultrasonore à la période de répétition des impulsions ultrasonore définit le rapport cyclique associé à la séquence d'émission. Typiquement les rapports cycliques mis en oeuvre sont de l'ordre de 50% c'est-à-dire que la durée d'émission de l'impulsion ultrasonore est de l'ordre de la durée d'attente.

Ainsi, afin de réduire le temps de traitement, une première catégorie de solutions consiste à augmenter le volume de la zone de nécrose en augmentant le volume de la zone focale et réduire ainsi le nombre d'impulsions ultrasonores requises pour couvrir le volume de traitement.

Pour augmenter le volume de la zone de nécrose, on peut augmenter la puissance de l'impulsion ultrasonore ou maintenir l'impulsion ultrasonore au-delà du temps nécessaire à la formation de la lésion dans le volume focal. La lésion biologique va alors s'étendre au-delà du volume focal particulièrement en direction de la sonde. Cette dynamique de formation de la lésion tissulaire permet de moduler l'extension de la zone nécrosée, mais elle a pour effet de sur-traiter la zone focale. Par ailleurs, le maintien de l'impulsion ultrasonore pendant des durées longues nécessitera, pour préserver les tissus intermédiaires, d'augmenter le temps d'attente entre chaque impulsion, c'est-à-dire de réduire le rapport cyclique, ce qui est contraire à l'objectif initial de réduction du temps de traitement.

Le document EP 1 274 483 propose d'augmenter le volume de la zone focale en augmentant le nombre d'ouverture de la sonde, c'est-à-dire le rapport de la distance focale au diamètre de la surface d'émission. Le diamètre de la surface d'émission est réduit en supprimant l'émission de groupes d'anneaux concentriques indépendants. Mais la réduction de l'ouverture de la sonde a pour effet d'augmenter l'intensité acoustique sur les tissus situés entre la zone focale et la sonde. Pour éviter de les léser, il convient alors de réduire l'énergie acoustique délivrée ou d'augmenter le temps d'attente entre chaque impulsion.

La technique appelée « split focus » est également connue pour augmenter le volume de la zone focale. Elle consiste à diviser la surface d'émission de la sonde ultrasonore en plusieurs secteurs alimentés en opposition de phase en vue de créer autant de points focaux que de secteurs individuels. L'intensité acoustique dans le volume focal est alors réduite et il convient d'augmenter l'énergie acoustique délivrée par la sonde pour conserver le même effet thérapeutique au risque de léser les tissus intermédiaires.

Le document EP 2 035 091 propose d'augmenter le volume focal en réalisant la sonde ultrasonore selon une géométrie torique. Cette géométrie de la sonde permet de former une double zone focale et d'augmenter de manière conséquente le volume de traitement. Cette solution se prête bien au traitement rapide de tumeurs de gros volume isolées dans un organe et ne nécessitant pas la préservation de tissus de voisinage, mais elle est moins bien adaptée au traitement précis de petits volumes.

La demande de brevet US 2011/144544 décrit un dispositif mécanique de déplacement du point focal du transducteur afin de balayer un large volume de tissus en un minimum de temps.

De même, la demande de brevet DE 694 17 639 décrit des moyens électroniques de déplacement du point focal pour traiter la totalité d'un volume d'une lésion.

Le brevet US 6 488 639 décrit un appareil de traitement par ultrasons focalisés, dont la fréquence est adaptée en fonction de la profondeur de la lésion à traiter en vue d'assurer une répartition optimale de l'énergie dans la cible.

La demande de brevet WO 2009/050719 concerne un appareil de traitement par ondes ultrasonores, décrivant notamment la loi d'atténuation des ondes acoustiques à travers un tissu traité.

Une deuxième catégorie de solutions consiste à réduire le temps d'attente entre chaque impulsion ultrasonore. Ainsi, le document FR 2 903 315 prévoit de supprimer le temps d'attente entre chaque impulsion ultrasonore en segmentant la surface d'émission en au moins deux parties alimentées indépendamment, alternativement et sensiblement consécutivement. Cette technique permet l'échauffement continu de la zone focale et également l'augmentation du volume focal par réduction de l'ouverture de la sonde. Mais il convient également de réduire l'énergie acoustique transmise par chacun des segments de la sonde pour éviter de léser les tissus intermédiaires. En conséquence, la même énergie est délivrée dans les tissus de manière continue mais sans réduction du temps de traitement.

La demande de brevet WO 2010/127369 prévoit également dans le domaine de traitement par sonoporation, de moduler le rapport cyclique en envisageant une émission continue.

Le brevet US 5 665 054 décrit une méthode reposant sur le déplacement rapide du point de focalisation d'un transducteur ultrasonore dans le plan focal afin d'obtenir une distribution de température constante dans un large volume. L'intensité ultrasonore nécessaire au maintien de cette température n'est pas constante dans le plan focal mais doit être plus importante en périphérie du volume focal afin de tenir compte des effets de la diffusion de la chaleur.

Le brevet US 5 665 054 prévoit de contrôler l'énergie délivrée aux tissus par modulation de la durée d'émission.

Dans le domaine du traitement des tissus du myocarde à l'aide d'un transducteur ultrasonore, la demande de brevet US 2013/0261461 décrit un appareil permettant de déplacer la zone focale pour traiter l'épaisseur du muscle. Cet appareil pilote l'arrêt de la durée d'émission des ondes ultrasonores dès qu'apparaît une zone hyperéchogène sur un moniteur de contrôle. Cet appareil permet de contrôler l'énergie délivrée aux tissus par modulation de la durée d'émission.

Afin de réduire le temps de traitement des thérapies ultrasonores focalisées, la publication «HIFU treatment time réduction in superficial tumours through focal zone path selection» Int. J. Hyperthermia, August 2011 ; 27(5) : 465-481, propose une étude sur l'optimisation des trajectoires des zones focales.

Différentes trajectoires sont étudiées comme des trajectoires axiales de type empilement de lésions puis déplacement latéral, des trajectoires planes correspondant à un déplacement latéral séquentiel et réalisation de plans de traitement successifs, des trajectoires circulaires pour chacun des plans ou des trajectoires 3D.

L'optimisation des trajectoires est réalisée en jouant à la fois sur les temps d'émission et les temps d'extinction de la sonde ultrasonore mais pas sur la puissance d'émission qui est maintenue constante pour chaque lésion. Ainsi, il est prévu l'application d'un temps d'émission jusqu'à atteindre la dose thermique, puis un temps d'extinction pendant une durée optimisée permettant le refroidissement des tissus d'interface. La dose thermique déposée antérieurement par les tirs précédents est retranchée de celle à atteindre. Par contre, la dose thermique issue des tirs suivants n'est pas prise en compte. Au total, il apparaît un surdosage.

Il ressort de cette étude que des traitements basés sur des trajectoires en empilement de lésions selon l'axe acoustique sont plus rapides que ceux fondés sur des déplacements latéraux. De même, il ressort de cette étude que le traitement est plus rapide lorsqu'il débute par le plan intermédiaire, par rapport à un traitement commençant par le plan distal. Par ailleurs, cette étude préconise d'augmenter la puissance émise par la sonde ultrasonore afin de réduire le temps de traitement.

Les simulations réalisées par cette étude concernent des volumes de traitement de taille réduite, inférieure à 5 cm$^3$. Pour des volumes de 5 cm$^3$, l'étude montre qu'il est nécessaire d'augmenter les temps d'extinction de l'émission ultrasonore jusqu'à atteindre 90% du temps de traitement, soit un rapport cyclique de 10%, pour permettre le refroidissement des tissus d'interface. La transposition à des volumes encore plus importants, typiquement de 30 à 40 cm$^3$, requiert des temps de traitement augmentés en proportion qui sont incompatibles avec un usage clinique.

La demande de brevet US 2013/041249 décrit un procédé de régulation de la dose thermique par modulation de l'énergie délivrée aux tissus. Cette modulation est obtenue par modulation de la durée d'émission puis si cette modulation est insuffisante en ajustant alors la puissance. Il est à noter que la réduction de la durée des tirs et la conservation de puissances élevées sont plutôt choisies pour avoir un effet biologique plus important et une extension mieux maitrisée. Ce document n'apporte pas une solution à la réduction du temps de traitement.

L'objet de l'invention vise à remédier aux inconvénients des solutions antérieures connues en proposant un nouvel appareil de traitement assurant l'application d'ondes ultrasonores focalisées sur une zone de traitement de grand volume, tout en permettant de réduire significativement la durée de traitement de ladite zone.

Pour atteindre un tel objectif, l'appareil pour le traitement thermique de tissus biologiques agit dans une zone de traitement, par l'application d'ondes ultrasonores focalisées, l'appareil comportant :

- une sonde ultrasonore délivrant une énergie ultrasonore focalisée dans une zone focale située à distance de la sonde ;
- un système de commande de la sonde ultrasonore pour déplacer la zone focale selon une trajectoire déterminée et pour piloter la puissance ultrasonore délivrée, sous la forme d'une répétition d'impulsions ultrasonores séparées chacune par un temps d'attente et présentant un temps d'émission dont le rapport à la période de répétition des impulsions ultrasonores définit le rapport cyclique. Selon l'invention, le système de commande comporte :

  ◦ un système de déplacement assurant :

  • le déplacement de la zone focale selon une première direction, d'un point distal de la zone de traitement à un point proximal de la zone de traitement, afin de juxtaposer des zones élémentaires de traitement et de réaliser une séquence de zones élémentaires de traitement ;
  • la juxtaposition des séquences de zones élémentaires de traitement selon au moins une deuxième direction différente de la première afin de couvrir complètement la zone de traitement ;

  ◦ un circuit de commande pilotant la sonde, afin que la puissance ultrasonore délivrée dans la zone focale diminue entre le point distal et le point proximal de la zone de traitement.

L'appareil selon l'invention comporte également en combinaison l'une et/ou l'autre des caractéristiques additionnelles suivantes :

- le circuit de commande pilote la sonde afin que le rapport cyclique des impulsions ultrasonores pour réaliser les séquences de zones élémentaires soit compris entre 65% et 100% et de préférence entre 85% et 100% ;

- le système de commande assure le déplacement de la zone focale selon l'axe acoustique en tant que première direction ;
- le système de commande comporte un système mécanique pour déplacer la sonde ultrasonore afin d'assurer le déplacement de la zone focale ;
- la sonde ultrasonore comporte une pluralité d'émetteurs ultrasonores et en ce que le système de commande comporte un système qui pilote électroniquement les émetteurs ultrasonores afin d'assurer le déplacement de la zone focale ;
- le système de commande pilote les émetteurs ultrasonores de telle manière que le « nombre d'ouverture » ou « nombre F » du transducteur, c'est-à-dire le rapport de la distance focale au diamètre de la sonde formée par les émetteurs ultrasonores activés, diminue lorsque la zone focale passe du point distal au point proximal ;
- le système de commande assure la diminution de la puissance ultrasonore émise selon une loi de correction $p_i$ telle que par exemple :

$$p_i = P_{ref} \exp(Ad_i)$$

Où :

  $p_i$ = puissance requise (Watts) pour une zone focale à la profondeur $d_i$ (cm).
  $P_{ref}$ = puissance de référence (Watts)
  A = coefficient d'atténuation (cm$^{-1}$).

Diverses autres caractéristiques ressortent de la description faite ci-dessous en référence aux dessins annexés qui montrent, à titre d'exemples non limitatifs, des formes de réalisation de l'objet de l'invention.

La **Figure 1** est une vue schématique d'un appareil pour le traitement thermique conforme à l'invention.
Les **Figures 1A à 1F** sont différentes vues de mise en oeuvre d'une première variante de réalisation d'un appareil de traitement conforme à l'invention.
La **Figure 2** est une vue schématique d'un autre exemple de réalisation d'un appareil de traitement conforme à l'invention.
Les **Figures 2A** et **2B** sont des vues illustrant les avantages pouvant être obtenus à l'aide d'un appareil de traitement décrit à la figure **2.**

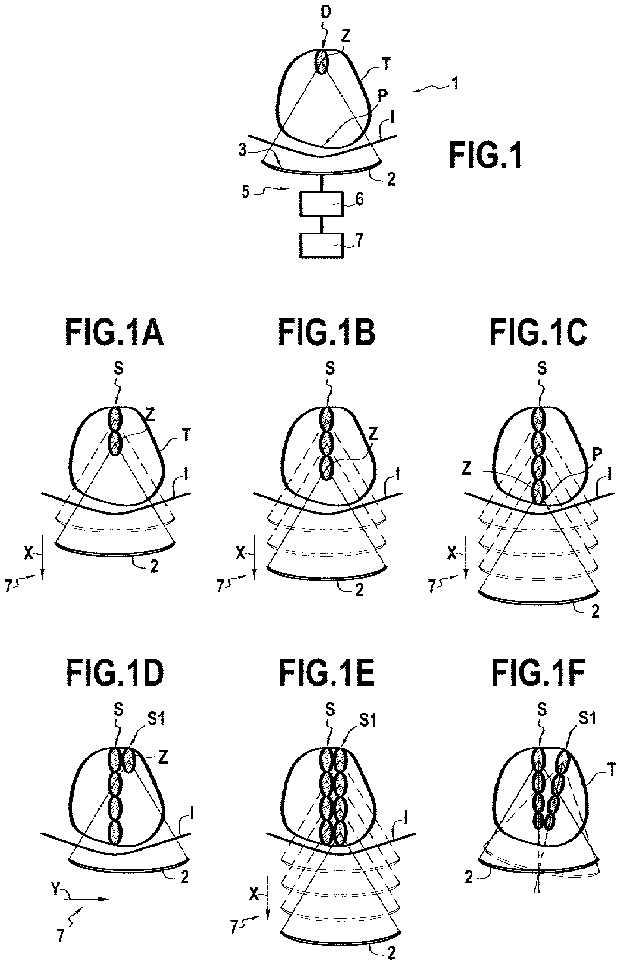

Tel que cela ressort plus précisément de la **Fig. 1,** l'objet de l'invention concerne un appareil **1** pour le traitement thermique de tissus biologiques dans une zone de traitement **T,** par l'application d'ultrasons focalisés de haute intensité (HIFU, High Intensity Focused Ultrasound). Cet appareil de traitement comporte classiquement, une sonde ultrasonore **2,** se présentant sous la forme d'un transducteur, comportant une face d'émission **3** d'ondes ultrasonores focalisées dans une zone focale **Z** correspondant à une zone élémentaire de traitement. La géométrie de cette zone focale **Z** dépend de la forme de la face d'émission. Classiquement, le transducteur comporte un ou plusieurs émetteurs ultrasonores tels que par exemple des éléments piézoélectriques.

De manière connue, cette sonde ultrasonore **2** est positionnée par abord extracorporel ou endocavitaire, à proximité de la zone d'interface **I** des tissus biologiques dont au moins une partie est à traiter. Dans la mesure où les tissus à traiter (c'est-à-dire la zone de traitement **T**) occupent un volume supérieur à la zone élémentaire de traitement (correspondant à la zone focale **Z**), alors la zone focale **Z** de la sonde ultrasonore **2** doit être déplacée selon une trajectoire déterminée pour traiter complètement les tissus de la zone de traitement **T**.

L'appareil **1** comporte également un système **5** de commande de la sonde ultrasonore **2** pour déplacer la zone focale **Z** selon une trajectoire déterminée et pour piloter l'énergie ultrasonore délivrée par la sonde ultrasonore. Un tel système de commande **5** comporte notamment un circuit de commande **6** délivrant des signaux pour activer les émetteurs ultrasonores via un étage d'amplification, et un système **7** pour déplacer la zone focale **Z** selon une trajectoire déterminée. Un tel circuit de commande **6** n'est pas décrit plus précisément car sa réalisation fait partie des connaissances techniques de l'homme du métier. Par ailleurs, comme cela sera décrit dans la suite de la description, le système **7** pour déplacer la zone focale **Z** est de nature « mécanique » et/ou « électronique ».

Conformément à l'invention, le système de déplacement **7** assure :

- le déplacement de la zone focale **Z** selon une première direction **X** d'un point distal **D** de la zone de traitement **T** à un point proximal **P** de la zone de traitement **T** afin de juxtaposer des zones élémentaires de traitement et de réaliser une séquence **S** de zones élémentaires de traitement ;
- la juxtaposition des séquences **S1**, **S2**, **S3**, ..., de zones élémentaires de traitement selon au moins une deuxième direction **Y** différente de la première **X** afin de couvrir complètement la zone de traitement **T**.

Le système de commande **5** pilote la sonde **2** à l'aide du circuit de commande **6** afin que la puissance ultrasonore délivrée dans la zone focale **Z** diminue entre le point distal **D** et le point proximal **P** de la zone de traitement. Selon une variante avantageuse de réalisation, le circuit de commande **6** est piloté de manière que le rapport cyclique des impulsions ultrasonores pour réaliser les séquences de zones élémentaires soit compris entre 65% et 100% et de préférence entre 85% et 100%. Il est rappelé que le circuit de commande **6** pilote la sonde ultrasonore de sorte que la puissance ultrasonore délivrée se présente sous la forme d'impulsions ultrasonores (ou tirs) séparées chacune par un temps d'attente. Le rapport du temps d'émission sur la période de répétition des impulsions ultrasonores définit le rapport cyclique.

Dans l'exemple de réalisation illustré aux **Fig. 1A à 1F**, le système de commande **5** comporte, en tant que système de déplacement **7,** un dispositif mécanique pour déplacer la sonde ultrasonore **2** afin d'assurer le déplacement de la zone focale **Z** pour couvrir toute la zone de traitement **T** selon les séquences de zones élémentaires et leurs juxtapositions. En d'autres termes, la sonde ultrasonore **2** est déplacée dans l'espace par tous systèmes mécaniques connus pour suivre la trajectoire décrite ci-dessus.

Selon l'exemple de réalisation illustré aux **Fig. 1A à 1E**, le système de commande **5** et plus précisément, le système de déplacement **7,** assure le déplacement de la zone focale **Z** selon une première direction **X** qui, de préférence, est confondue avec l'axe acoustique de la sonde ultrasonore. Le système de commande **5** positionne ainsi la zone focale **Z** en sa position la plus profonde ou la plus éloignée par rapport à la sonde ultrasonore **2**. Dans cette position, illustrée à la **Fig. 1,** le système de commande **5** et plus précisément le circuit de commande **6** est piloté pour réaliser la lésion élémentaire à une puissance $P_i$, avec une durée de l'impulsion ultrasonore égale par exemple à une seconde. Puis, à l'aide du système de déplacement **7,** la sonde ultrasonore **2** est reculée physiquement afin de positionner la zone focale **Z** dans une deuxième position entraînant une seconde lésion élémentaire juxtaposée à la précédente (**Fig. 1A**). La juxtaposition de la seconde lésion par rapport à la première doit permettre un traitement homogène de la zone de traitement **T**, c'est dire sans sur-traitement ou sous-traitement. Pour ce faire les lésions peuvent être juxtaposées avec ou sans recouvrement des unes par rapport aux autres. Dans cette deuxième position de la zone focale **Z**, la sonde ultrasonore **2** émet une impulsion ultrasonore dans un délai minimal par rapport à la réalisation de la première zone focale.

La seconde lésion élémentaire devant être réalisée à une profondeur plus faible, la puissance de l'impulsion ultrasonore est réduite afin de conserver le même volume de lésion élémentaire. La loi de correction de la puissance est de type $p_i$ telle que par exemple :

$$p_i = P_{ref}\, exp(Ad_i)$$

Où:

$p_i$ = puissance requise (Watts) pour une zone focale à la profondeur $d_i$ (cm).
$P_{ref}$ = puissance de référence (Watts)
$A$ = coefficient d'atténuation (cm$^{-1}$).

Le coefficient d'atténuation A est caractéristique du tissu traité, de la fréquence des ondes ultrasonores et des conditions de traitement. Par exemple un tissu préalablement échauffé ou dans lequel des bulles de cavitation se sont formées présentera un coefficient d'atténuation différent de celui initial.

La loi de correction de la puissance donnée en exemple est particulièrement adaptée au traitement de gros volumes situés à une distance importante des interfaces et nécessitant la juxtaposition de plusieurs zones focales élémentaires. Pour des lésions moins profondes, la loi de correction de puissance peut prendre la forme plus simple d'une fonction linéaire. L'essentiel est que cette loi de correction de puissance induise une diminution de la puissance entre les impulsions ultrasonores réalisées en zone distale et celles réalisées en zone proximale.

Comme cela ressort plus précisément des **Fig. 1B et 1C,** le système de commande **5** répète les opérations de déplacement de la zone focale **Z** selon la première direction **X,** du point distal **D** au point proximal **P,** et les opérations d'émission des impulsions ultrasonores, avec d'une part une puissance qui diminue entre le point distal **D** et le point proximal **P** et d'autre part, selon une variante avantageuse de réalisation, le rapport cyclique des impulsions ultrasonores pour réaliser les séquences de zones élémentaires qui soit compris entre 65% et 100% et de préférence entre 85% et 100%.

Le déplacement de la zone focale **Z** selon la première direction **X** du point distal **D** au point proximal **P** permet de réaliser une séquence de une à plusieurs zones élémentaires de traitement (au nombre de 4 dans l'exemple illustré). Bien entendu, pour une trajectoire donnée selon la première direction **X,** le nombre de lésions élémentaires peut être augmenté à « l'infini » en réduisant le pas entre chacune d'elles pour tendre vers un traitement spatialement continu dans lequel la puissance peut être ajustée de manière continue selon la loi de correction pour tendre également vers une modulation temporelle continue de la puissance.

Comme expliqué ci-dessus, le système de commande **5** assure le déplacement de la zone focale **Z** du point distal **D** au point proximal **P,** avec une puissance ultrasonore qui diminue entre le point distal et le point proximal de la zone de traitement. Ainsi, la puissance acoustique requise pour former chacune de ces lésions élémentaires est réduite à mesure que les lésions élémentaires s'approchent de la zone d'interface **I.** Les puissances acoustiques étant de plus en plus faibles, les tissus d'interface sont de moins en moins exposés à un dépôt de chaleur et la vascularisation naturelle devient suffisante pour dissiper ce dépôt de chaleur. Dès lors, le temps d'attente avant de lancer la séquence de tir qui permettra de juxtaposer le volume de lésion élémentaire suivant peut être minimisé.

Typiquement, l'appareil **1** selon l'invention permet de réaliser une séquence par exemple de 4 zones élémentaires de traitement en 4 secondes. Les quatre impulsions ultrasonores durent chacune 1 seconde et se suivent sans délai d'attente. Le rapport cyclique sur cette séquence est ainsi proche de 100%. Le délai d'attente varie selon que le système **7** pour déplacer et juxtaposer les zones focales **Z** et les séquences de zones élémentaires de traitement est de nature « mécanique » et / ou « électronique ». Si le système **7** est de nature électronique, le délai d'attente est négligeable par rapport à la durée d'émission et le rapport cyclique approche 100%. Si le système **7** est de nature mécanique, le délai d'attente est sensiblement augmenté pour permettre le déplacement de la sonde par les moyens mécaniques, il peut être de l'ordre de 200 ms voire 500 ms environ, réduisant alors le rapport cyclique à 85% voire 65% environ.

Dans l'art antérieur, un délai d'attente de 4 s après une impulsion de tir de 4 s, doit être respecté avant l'impulsion de tir suivante pour permettre le refroidissement des tissus d'interface. Le rapport cyclique est ainsi de 50%. A la différence des techniques antérieures qui imposent un délai d'attente entre la réalisation de chaque zone élémentaire de traitement ou un délai d'attente entre la réalisation de chaque séquence de zones élémentaires, le traitement réalisé avec l'appareil **1** selon l'invention se déroule avec des délais d'attente qui peuvent être réduits au minimum voire quasi nuls permettant des séquences de traitement avec des rapports cycliques proche de 100%, ou 85% à 65% selon les systèmes de déplacement. Ces rapports cycliques permettent de réduire la durée des traitements de manière sensible par rapport aux techniques antérieures. Dans l'art antérieur, par exemple, le traitement d'une zone de traitement **T** nécessitant la répétition de 500 lésions avec un rapport cyclique de 50% basé sur 5 secondes d'émission et 5 secondes d'attente durera 80 minutes environ. Selon l'invention le même traitement conduit avec des séquences de tirs basées sur un rapport cyclique de 85% sera conduit en 50 minutes. A titre d'exemple, le volume de la zone traitement **T** est par exemple compris entre 25 et 35 cm$^3$.

Le système de commande **5** assure, par le système de déplacement **7,** dans l'exemple illustré aux **Fig. 1D et 1E,** le déplacement de la sonde ultrasonore **2** selon la direction **Y** pour réaliser de manière juxtaposée et avec un délai minimal, une ou plusieurs séquences de zones élémentaires de traitement, **S1, S2, S3,** ..., afin de couvrir complètement la zone de traitement. Le déplacement de la sonde ultrasonore **2** selon la direction **Y** peut être obtenu par translation (**Fig. 1D** et **1E**) ou par rotation permettant des trajectoires sectorielles (**Fig. 1F**). Bien entendu, la sonde ultrasonore peut être déplacée selon une troisième direction pour couvrir tout un volume.

Conformément à l'invention, chacune des séquences de zones élémentaires de traitement **S1, S2, S3,** ..., est réalisée selon le principe décrit en relation des **Fig. 1 à 1C.** Ainsi, pour chacune des séquences de zones élémentaires de traitement **S1, S2, S3,** ..., la puissance ultrasonore délivrée dans la zone focale **Z** diminue entre le point distal **D** et le point proximal **P** de la zone de traitement. De plus, selon une variante avantageuse de réalisation, le rapport cyclique des impulsions ultrasonores pour réaliser les séquences de zones élémentaires de traitement **S1**, **S2, S3**, ..., est compris entre 65% et 100% et de préférence entre 85% et 100%. Bien entendu, chacune des séquences de zones élémentaires de traitement **S1, S2, S3**, ..., comporte un nombre de zones élémentaires de traitement identique ou différent. La réalisation successive de ces séquences, avec un délai minimal entre chacune d'elles, permet de limiter également le temps de traitement.

Il est à noter que dans le cadre d'un traitement endocavitaire, le recul physique de la sonde ultrasonore **2** est souvent limité par des considérations anatomiques.

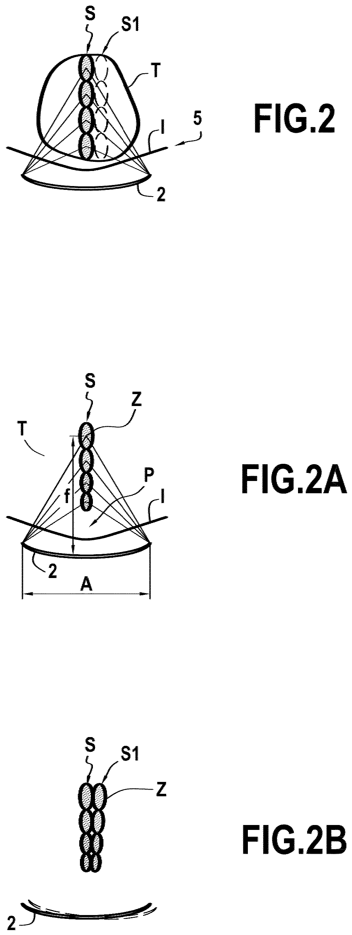

La **Fig. 2** illustre un autre exemple de réalisation dans lequel le système de commande **5** comporte en tant que système de déplacement **7,** un système pilotant électroniquement les émetteurs ultrasonores de la sonde afin d'assurer, moyennant une loi de retard, le déplacement selon la direction **X,** de la zone focale **Z** de la sonde ultrasonore **2** sans déplacement mécanique.

Le déplacement de la zone focale **Z** de la sonde ultrasonore est réalisé par des moyens électroniques **7** connus en soi. Une telle solution consiste à disposer d'une sonde présentant une multitude d'émetteurs ultrasonores répartis par exemple selon une géométrie sphérique permettant de diviser la surface d'émission **3** de la sonde ultrasonore **2** par exemple en plusieurs anneaux concentriques indépendants, chacun étant alimenté par un signal électrique indépendant permettant d'appliquer des lois de retard entre chacun des signaux. Ces lois de retard permettent de modifier la position du point de convergence du champ ultrasonore le long de l'axe acoustique de la sonde ultrasonore, sans déplacement physique de la sonde ultrasonore. La loi de puissance décrite précédemment est appliquée à l'aide du circuit de commande **6,** lors de la réalisation de la juxtaposition des lésions élémentaires nécessaires à l'obtention d'une séquence **S** de zones élémentaires de traitement. De manière similaire, le passage de la séquence **S** de zones élémentaires de traitement à la séquence **S1** de zones élémentaires de traitement peut se faire à l'aide d'un système de déplacement **7** de nature mécanique ou électronique. Les séquences **S1**, **S2, S3,** ... de zones élémentaires de traitement sont juxtaposées avec un délai minimal comme expliqué précédemment.

Cette solution présente en outre l'avantage de pouvoir réaliser les lésions de proximité avec une configuration géométrique de la sonde ultrasonore pour laquelle le nombre d'ouverture ou nombre **F** défini comme le rapport de la distance focale au diamètre de la sonde formée par les émetteurs ultrasonores activés, diminue à mesure que les lésions élémentaires sont réalisées à proximité de la sonde ultrasonore **2**. La diminution du nombre d'ouverture de la sonde ultrasonore **2** permet une meilleure focalisation du champ ultrasonore et une meilleure préservation des tissus intermédiaires en réduisant l'intensité acoustique ultrasonore sur la zone d'interface **I**. La meilleure focalisation du champ ultrasonore permet également d'obtenir la même intensité acoustique au point de focalisation avec une puissance acoustique réduite permettant encore la préservation des tissus intermédiaires. La meilleure focalisation permet également de réduire le volume de chaque zone focale **Z** à mesure que celles-ci se rapprochent du point proximal **P** de la zone de traitement **T** comme illustré sur la figure **2A**.

La réduction du volume des zones focales proximales permet une meilleure juxtaposition des séquences de lésions élémentaires **S** et **S1** lors de la réalisation de trajectoires sectorielles comme décrites en figure 2B. Cette solution, en réduisant le recouvrement des zones focales **Z** en zone proximale, évite la surexposition des zones proximales et des tissus intermédiaires tout en permettant la juxtaposition des zones focales **Z** en zone distale.

L'invention n'est pas limitée aux exemples décrits et représentés car diverses modifications peuvent y être apportées sans sortir de son cadre.

**Revendications**

**1.** Appareil pour le traitement thermique de tissus biologiques dans une zone de traitement (**T**), par l'application d'ondes ultrasonores focalisées, l'appareil comportant :

  - une sonde ultrasonore (**2**) délivrant une énergie ultrasonore focalisée dans une zone focale (**Z**) située à distance de la sonde ;

  un système de commande (**5**) de la sonde ultrasonore (**2**) comportant un circuit de commande (**6**) pour piloter la puissance ultrasonore délivrée, sous la forme d'une répétition d'impulsions ultrasonores séparées chacune par un temps d'attente et présentant un temps d'émission dont le rapport à la période de répétition des impulsions ultrasonores définit le rapport cyclique, ce système de commande (**5**) comportant un système de déplacement (**7**) assurant le déplacement de la zone focale (**Z**) selon une première direction (**X**) afin de juxtaposer des zones élémentaires de traitement et de réaliser une séquence (**S**) de zones élémentaires de traitement et selon au moins une deuxième direction (**Y**) différente de la première (**X**) afin de couvrir complètement la zone de traitement (**T**) ;
  **caractérisé en ce que** le système de déplacement (**7**) assure d'une part le déplacement de la zone focale (**Z**) d'un point distal (**D**) de la zone de traitement (**T**) à un point proximal (**P**) de la zone de traitement pour réaliser la séquence (**S**) de zones élémentaires de traitement selon la première direction, et d'autre part, la juxtaposition selon la deuxième direction (**Y**), de séquences (**S, S₁, S₂, S₃,** ...) de zones élémentaires de traitement juxtaposées selon la première direction (**X**), avec pour chacune desdites séquences, le déplacement de la zone focale (**Z**) d'un point distal (**D**) de la zone de traitement (**T**) à un point proximal (**P**) de la zone de traitement, et **en ce que** le circuit de commande (**6**) pilote la sonde (**2**), afin que la puissance ultrasonore délivrée dans la zone focale diminue entre le point distal (**D**) et le point proximal (**P**) de la zone de traitement.

2. Appareil selon la revendication 1, **caractérisé en ce que** le circuit de commande (**6**) délivre des signaux pour activer la sonde (**2**), afin que le rapport cyclique des impulsions ultrasonores délivrées par la sonde (**2**) pour réaliser les séquences de zones élémentaires soit compris entre 65% et 100% et de préférence entre 85% et 100%.

3. Appareil selon l'une des revendications 1 ou 2, **caractérisé en ce que** le système de commande (**5**) comporte un système (**7**) pour déplacer la zone focale (**Z**) selon l'axe acoustique en tant que première direction (**X**).

4. Appareil selon l'une des revendications 1 à 3, **caractérisé en ce que** le système de commande (**5**) comporte un système mécanique (**7**) pour déplacer la sonde ultrasonore (**2**) afin d'assurer le déplacement de la zone focale.

5. Appareil selon l'une des revendications 1 à 4, **caractérisé en ce que** la sonde ultrasonore (**2**) comporte une pluralité d'émetteurs ultrasonores et **en ce que** le système de commande (**5**) comporte un système (**7**) qui pilote électroniquement les émetteurs ultrasonores afin d'assurer le déplacement de la zone focale (**Z**).

6. Appareil selon la revendication 5, **caractérisé en ce que** le système de commande (**5**) délivre des signaux pour activer les émetteurs ultrasonores de manière que le nombre d'ouverture de la sonde, c'est à dire le rapport de la distance focale au diamètre de la sonde (**2**) formée par les émetteurs ultrasonores activés, diminue lorsque la zone focale (**Z**) passe du point distal (**D**) au point proximal (**P**).

7. Appareil selon l'une des revendications 1 à 6, **caractérisé en ce que** le système de commande (**5**) délivre des signaux pour activer les émetteurs ultrasonores pour assurer la diminution de l'énergie ultrasonore émise selon une loi de correction $p_i$ telle que :

$$p_i = P_{ref} \exp(Ad_i)$$

Où :

$p_i$ = puissance requise (Watts) pour une zone focale à la profondeur $d_i$ (cm).
$P_{ref}$ = puissance de référence (Watts)
$A$ = coefficient d'atténuation (cm⁻¹).

**Patentansprüche**

1. Vorrichtung zur Wärmebehandlung biologischer Gewebe in einem Behandlungsbereich (T) durch das Anwenden von fokussierten Ultraschallwellen, wobei die Vorrichtung aufweist:

   - eine Ultraschallsonde (2), die eine fokussierte Ultraschallenergie in einen Fokalbereich (Z) abgibt, der in einem Abstand von der Sonde angeordnet ist,

   ein Steuersystem (5) der Ultraschallsonde (2), umfassend eine Steuerschaltung (6) zum Steuern der abgegebenen Ultraschallleistung in der Form von einer Wiederholung von Ultraschallimpulsen, die jeweils durch eine Wartezeit getrennt sind und eine Sendezeit aufweisen, deren Verhältnis zu der Wiederholperiode der Ultraschallimpulse das zyklische Verhältnis definiert, wobei dieses Steuersystem (5) ein Verschiebungssystem (7) aufweist, das das Verschieben des Fokalbereichs (Z) in einer ersten Richtung (X), um elementare Behandlungsbereiche aneinander zu reihen und eine Sequenz (S) von elementaren Behandlungsbereichen zu erstellen, und in mindestens einer zweiten Richtung (Y) gewährleistet, die von der ersten (X) verschieden ist, um den Behandlungsbereich (T) vollständig zu bedecken,
   **dadurch gekennzeichnet, dass** das Verschiebungssystem (7) einerseits das Verschieben des Fokalbereichs (Z) von einem distalen Punkt (D) des Behandlungsbereichs (T) zu einem proximalen Punkt (P) des Behandlungsbereichs, um die Sequenz (S) von elementaren Behandlungsbereichen in der ersten Richtung zu erstellen, und andererseits das Aneinanderreihen in der zweiten Richtung (Y) von Sequenzen (S, $S_1$, $S_2$, $S_3$, ...) elementarer Behandlungszonen, die in der ersten Richtung (X) aneinandergereiht sind, mit dem Verschieben für jede der Sequenzen des Fokalbereichs (Z) von einem distalen Punkt (D) des Behandlungsbereichs (T) zu einem proximalen Punkt (P) des Behandlungsbereichs gewährleistet, und dadurch, dass die Steuerschaltung (6) die Sonde (2) steuert, damit die Ultraschallleistung, die in den Fokalbereich abgegeben wird, zwischen dem distalen Punkt (D) und dem proximalen Punkt (P) des Behandlungsbereichs abnimmt.

2. Vorrichtung gemäß Anspruch 1, **dadurch gekennzeichnet, dass** die Steuerschaltung (6) Signale abgibt, um die Sonde (2) zu aktivieren, damit das zyklische Verhältnis der Ultraschallimpulse, die von der Sonde (2) abgegeben werden, um die Sequenzen von elementaren Behandlungsbereichen zu erstellen, zwischen 65 % und 100 %, vorzugsweise 85 % und 100 % beträgt.

3. Vorrichtung gemäß einem der Ansprüche 1 oder 2, **dadurch gekennzeichnet, dass** das Steuersystem (5) ein System (7) aufweist, um den Fokalbereich (Z) entlang der akustischen Achse als die erste Richtung (X) zu verschieben.

4. Vorrichtung gemäß einem der Ansprüche 1 bis 3, **dadurch gekennzeichnet, dass** das Steuersystem (5) ein mechanisches System (7) aufweist, um die Ultraschallsonde (2) zu verschieben, um das Verschieben des Fokalbereichs zu gewährleisten.

5. Vorrichtung gemäß einem der Ansprüche 1 bis 4, **dadurch gekennzeichnet, dass** die Ultraschallsonde (2) mehrere Ultraschallsender aufweist und dadurch, dass das Steuersystem (5) ein System (7) aufweist, das die Ultraschallsender elektronisch steuert, um das Verschieben des Fokalbereichs (Z) zu gewährleisten.

6. Vorrichtung gemäß Anspruch 5, **dadurch gekennzeichnet, dass** die Steuerschaltung (5) Signale abgibt, um die Ultraschallsender derart zu aktivieren, dass die Öffnungsanzahl der Sonde, das heißt das Verhältnis der Brennweite zu dem Durchmesser der Sonde (2), die durch die aktivierten Ultraschallsender gebildet ist, abnimmt, wenn der Fokalbereich (Z) von dem distalen Punkt (D) zu dem proximalen Punkt (P) übergeht.

7. Vorrichtung gemäß einem der Ansprüche 1 bis 6, **dadurch gekennzeichnet, dass** das Steuersystem (5) Signale abgibt, um die Ultraschallsender zu aktivieren, um die Verringerung der abgegebenen Ultraschallenergie nach
einem derartigen Korrekturgesetz $p_i$ zu gewährleisten, dass:

$$p_i = P_{ref}\, exp(Ad_i),$$

mit:

$p_i$ = erforderliche Leistung (Watt) für einen Fokalbereich in der Tiefe $d_i$ (cm),
$P_{ref}$ = Bezugsleistung (Watt),
$A$ = Dämpfungskoeffizient (cm$^{-1}$).

**Claims**

1. An apparatus for the thermal treatment of biological tissues in a treatment zone **(T),** by means of the application of focused ultrasound waves, the apparatus comprising :

   - an ultrasound probe **(2)** delivering focused ultrasound energy in a focal zone **(Z)** located at a distance from the probe;

   a control system **(5)** for controlling the ultrasound probe **(2)** comprising a control circuit **(6)** to control the delivered ultrasonic power, in the form of a repetition of ultrasonic pulses, each being separated by a waiting time and having an emission time whose ratio to the period of repetition of ultrasonic pulses defines the duty cycle, this control system **(5)** comprising a movement system **(7)** ensuring the moving of the focal zone **(Z)** along a first direction **(X)** in order to juxtapose the elementary treatment zones and to create a sequence **(S)** of elementary treatment zones along at least one second direction **(Y)** that is different from the first direction **(X),** in order to completely cover the treatment zone **(T);**
   **characterized in that** the movement system **(7)** ensures on the one hand the moving of the focal zone **(Z)** from a distal point **(D)** of the treatment zone **(T)** to a proximal point **(P)** of the treatment zone to create the sequence **(S)** of elementary treatment zones along the first direction, and on the other hand, the juxtaposition along the second direction **(Y),** of sequences **(S, S$_1$, S$_2$, S$_3$, ...)** of elementary treatment zones along the first direction **(X),** with for each of said sequences, the moving of the focal zone **(Z)** from a distal point **(D)** of the treatment zone **(T)** to a proximal point **(P)** of the treatment zone, and **in that** the control circuit **(6)** controls the probe **(2),** so that the ultrasonic power delivered in the focal zone decreases between the distal point **(D)** and the proximal point **(P)** of the treatment zone.

2. The apparatus according to claim 1, **characterized in that** the control circuit **(6)** delivers signals for activating the probe **(2),** so as to ensure that the duty cycle ratio of the ultrasonic pulses delivered by the probe **(2)** for creating the sequences of elementary zones is comprised between 65% and 100% and preferably between 85% and 100%.

3. The apparatus according to one of claims 1 or 2, **characterized in that** the control system **(5)** includes a system **(7)** for moving the focal zone **(Z)** along the acoustic axis that serves as a first direction **(X).**

4. The apparatus according to one of claims 1 to 3, **characterized in that** the control system **(5)** includes a mechanical system **(7)** for moving the ultrasound probe **(2)** in order to ensure the movement of the focal zone.

5. The apparatus according to one of claims 1 to 4, **characterized in that** the ultrasound probe **(2)** comprises a plurality of ultrasound emitters and **in that** the control system **(5)** includes a system **(7)** which electronically controls the ultrasound emitters in order to ensure the movement of the focal zone **(Z).**

6. The apparatus according to claim 5, **characterized in that** the control system **(5)** outputs signals to activate the ultrasound emitters in a manner such that the aperture number of the probe, that is to say the ratio of the focal length to the diameter of the probe **(2)** formed by the activated ultrasound emitters, decreases when the focal zone **(Z)** passes from the distal point **(D)** to the proximal point **(P).**

7. The apparatus according to one of claims 1 to 6, **characterized in that** the control system **(5)** outputs signals to activate the ultrasound emitters in order to ensure the reduction of the ultrasonic power emitted in accordance with a correction law $p_i$ such as :

$$p_i = P_{ref} \exp (Ad_i)$$

Where:

$p_i$ = power required (Watts) for a focal zone at the depth $d_i$ (cm).
$P_{ref}$ = reference power (Watts)
A = attenuation coefficient (cm$^{-1}$).

**FIG.1**

**FIG.1A**

**FIG.1B**

**FIG.1C**

**FIG.1D**

**FIG.1E**

**FIG.1F**

FIG.2

FIG.2A

FIG.2B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 1858591 A
- EP 1274483 A
- EP 2035091 A
- US 2011144544 A
- DE 69417639
- US 6488639 B
- WO 2009050719 A
- FR 2903315
- WO 2010127369 A
- US 5665054 A
- US 20130261461 A
- US 2013041249 A

### Littérature non-brevet citée dans la description

- HIFU treatment time réduction in superficial tumours through focal zone path selection. *Int. J. Hyperthermia,* Août 2011, vol. 27 (5), 465-481